# EUROPEAN PATENT APPLICATION

(11) **EP 2 471 574 A1**
(43) Date of publication of application: **04.07.2012**
(21) Application number: 11150089.8
(22) Date of filing: 04.01.2011
(51) Int. Cl.: A61M 39/10, A61M 16/08

(54) **Connector structure and a sampling tube of a patient respiratory tubing**

(71) Applicant: General Electric Company, Schenectady, NY 12345 (US)
(72) Inventor: Ranta, Janne, 02710, Espoo (FI); Holopainen, Timo, 00510 Helsinski (FI); Viitala, Juha, 01710 Vantaa (FI); Hakanen, Jukka, 02750 Espoo (FI)
(74) Representative: Valkeiskangas, Tapio Lassi Paavali

(57) **Abstract**

Connector structure for a fluid tube the connector structure comprising a first connector body (13) connectable to a fluid tube (6), and a second connector body (14). The first connector body and the second connector body are adapted to be connected to each other and provided with mating surfaces (16, 17) to create a fluid tight seal between the first connector body (13) and the second connector body (14). The mating surfaces (16, 17) comprise only vertical and/or oblique parts and the connector structure is provided with a locking mechanism (18) and that the mating surfaces (16, 17) have been arranged to press tightly against each other and form a fluid tight seal with the locking mechanism (18) and the first and the second connector body (13, 14) are arranged to detach from each other without correct use of the locking mechanism (18). The connector structure may be used in a sampling tube of a patient respiratory gas tubing.

## Description

### BACKGROUND OF THE INVENTION

The disclosure relates to a connector structure for a fluid tube the connector structure comprising a first connector body connected to a fluid tube, and a second connector body, the first connector body and the second connector body being adapted to be connected to each other and provided with mating surfaces to create a fluid tight seal between the first connector body and the second connector body. The disclosure relates further to a patient respiratory tubing using the connector structure.

As described above the disclosure relates to the connector structure for a fluid tube. More specifically the disclosure relates generally to a fluid connection for reducing the risk of incomplete connections resulting in leakages of fluid said connection. The disclosure relates also to sample tubing connections used in analyzing equipment such as gas analyzing equipment for patient respiratory gas.

In anesthesia or in intensive care, the condition of a patient is often monitored e.g. by analyzing the air exhaled by the patient for its carbon dioxide content. For this reason a small portion of the respiratory gas is delivered to a gas analyzer. The sample is carried along a sampling tube connected in one end often to a respiratory tube adapter and the other end to the gas analyzer. This sampling tube is typically disposable and must have some kind of reliable and tight connectors. Almost all pneumatic connectors in the respiratory system have tapered conical contact surfaces. Such connectors are simple, relatively easy to connect and cheap to make. The connection such as a well-known fitting called Luer-Lok, a registered trademark of Becton Dickinson of Franklin Lakes, NJ USA, has been in general use for gas sampling but also other similar connectors with differing dimensions can be used. When used with carefully following the instructions for use they provide an airtight and reliable connection.

A gas analyzer designed to measure respiratory gas in real time provides critical data that care givers use to assess the clinical status of the patient as well as the proper functioning of the devices and the clinical set up in use. The technology used in clinical gas measurement is developing into direction where the monitoring system is equipped with algorithms that provide caregivers proposals to readjust the clinical parameters based on the gas data. Therefore, it is of paramount importance that the data provided by the gas analyzer is correct and gives accurate and true information about the gas concentrations and their changes in the clinical set up.

Unfortunately, the connector designs used in gas sampling applications, including the widely used Luer connector, leave room for erroneous situations that can degrade the gas sample and lead into wrong clinical assessments. Specifically, it is possible to make a connection where the interfacing parts are mounted deep enough to each other so that they would not fall off from each other even thought still not being secured together. Often times such an incomplete and unsecured connection is not airtight even thought the gas sampling line hanging in gas detector gives a care giver the false visual impression that a proper connection has established.

Since the analyzer creates an under pressure into the gas sample line a leak in the connection will result in ambient air diluting the gas sample going into the analyzer meaning that the air analyzed in the gas sensor no longer represents the real clinical conditions. This can lead into wrong diagnoses about the patient's clinical condition as well as wrong conclusions about the functioning of the used equipment and their accessories. Specifically, patient safety may be worsened because incorrect gas data may lead to hypoxia or over dosage of anesthetic agent.

### BRIEF DESCRIPTION OF THE INVENTION

The above-mentioned shortcomings, disadvantages and problems herein which will be understood by reading and understanding the following specification.

In an embodiment, connector structure for a fluid tube the connector structure comprising a first connector body connected to a fluid tube, and a second connector body. The first connector body and the second connector body are adapted to be connectable to each other and provided with mating surfaces to create a fluid tight seal between the first connector body and the second connector body. The mating surfaces comprise only vertical and/or oblique parts and the connector structure is provided with a locking mechanism and that the mating surfaces have been arranged to press tightly against each other and form a fluid tight seal with the locking mechanism. The first and the second connector body are arranged to detach from each other without correct use of the locking mechanism.

In another embodiment in a sample tube of a patient respiratory gas tubing, at least one end of the sample tube is provided with the first connector body of the connection structure.

In a still another embodiment in a sample tube of a patient respiratory gas tubing, at least one end of the sample tube is provided with the second connector bodyof the connection structure.

### BRIEF DESCRIPTION OF THE DRAWINGS

Figure 1 shows a perspective view of the embodiment in an operating patient care environment,
Figure 3 shows schematically a first embodiment,
Figure 4 shows schematically a second embodiment,
Figure 5 shows schematically a third embodiment,
Figure 6 shows schematically a fourth embodiment,
Figure 7 shows schematically a fifth embodiment,
Figure 8 shows schematically a sixth embodiment,
Figure 9 shows schematically a seventh embodiment in fallen off situation, and
Figure 10 shows schematically a seveth embodiment in normal operating situation.

### DETAILED DESCRIPTION OF THE INVENTION

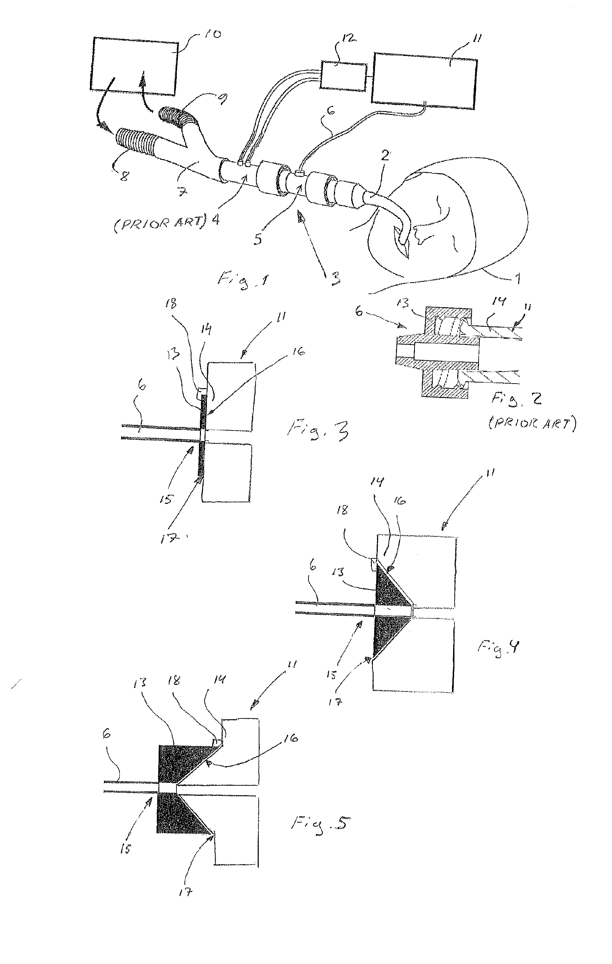

Figure 1 shows a normal operating patient care environment. In the arrangement shown in Figure 1 reference number 1 shows a patient and reference number 2 shows an intubation tube. Reference number 3 shows generally a patient breathing tubing. The patient breathing tubing comprises a flow sensor 4 and a connecting piece 5. The connecting piece 5 is provided with a sampling tube 6 for the analysis of gas, e.g. measurement of its concentration, is connected between the intubation tube and an Y-piece 7 connecting the inlet and outlet hoses 8, 9 of an apparatus 10 maintaining respiration.

The gas sampling tube 6 is connected to an analyzer 11, in which the gas is measured and the signal is processed so as to produce a display (not shown) showing the variations in the gas concentration under measurement as a function of time, i.e. the respiration curve or concentration readings-during exhalation and inhalation.

In the arrangement of Figure 1 the flow sensor 4 is also connected via measuring apparatus 12 to the analyzer 11, in which the signal is processed as to produce a display (not shown) of the flow and pressure readings for inhalation and exhalation and possible other quantities derived from them. The measuring apparatus 12 may also be placed in the analyzer 11 and the gas concentration can be performed in the connecting piece 5 or as integrated with the flow sensor 4, if the device is a so-called mainstream gas sensor.

When the gas sampling tube 6 is connected to the analyzer 11 in the prior art it is quite possible that there may exist disadvantages, i.e. eventual leaks etc. discussed earlier in the text.

Figure 2 shows a typical example of the prior art connector structure. Figure 2 uses corresponding refeence numbers as used in Figure 1. Figure 2 shows a connector using mating tapered conical contact surfaces. This prior art has been discussed earlier in the text.

The matters described above are quite familiar to a person skilled in the art and therefore said matters are not discussed here in detail.

Figure 3 shows the first embodiment. Figure 2 shows schematically the connection of the sampling tube 6 to the analyzer 11.

The embodiment shown comprises a first connector body 13 and a second connector body 14. The first connector body 13 comprises an element 15 for fastening the first connector body to the fluid tube 6, for example a gas sampling tube as shown in Figure 1. The second connector body 14 is provided with mating surfaces 16, 17 to create a fluid tight seal between the first connector body 13 and the second connector body 14.

The first connector body 13 and the second connector body 14 may be separate elements so that for example the second connector body 14 may be attaced to the analyzer 11. It is however also possible that said second connector body is an integral part of the analyzer 11 etc.

As described earlier there exist problems in the pror art. Said problems can however be solved with special connector geometries using special mating surfaces that significantly reduce the likelihood of generating leaking gas sample connections and virtually make those impossible. This may be accomplished with a geometry where the gas sampling line detaches from the gas detector unless it is appropriately attached and secured. The geometry offers no support for the sampling line to stay on the gas analyzer interface and does not provide a seemingly safe connection unless the mating parts are secured by using for example an approriate locking mechanism. This can be generated by having mating parts extending into each other as little as possible before secured and having vertical or appropriately tilted interfacing surfaces so that one connector part falls off for example because of gravity force.

The mating surfaces 16, 17 comprise only vertical and/or oblique parts and the connector structure is provided with a locking mechanism 18. The mating surfaces 16, 17 have been arranged to press tightly against each other and form a fluid tight seal with the locking mechanism 18 only. The first connector body 13 is arranged to detach from the second connector body 14 without correct use of the locking mechanism 18, i.e. if the locking mechanism 18 is not properly locked gravity force acting on the first connector body 13 in Figure 3 forces the first connector body 1 to detach.

The embodiment described above can also be described by desribing the matters in opposite way, i.e. by describing a typical prior art design in which a narrow mating connector protrudes into a female connector and the friction between the surfaces together with gravity can generate an illusion about a secured and hence airtight connection.

In the embodiment of Figure 3 the mating surfaces 16, 17 are flat surfaces. This is not however the only possibility but the mating surfaces may have other forms too.

Figure 4 shows schematically a second embodiment. Figure 4 uses corresponding reference numbers as used in Figures 1 and 2.

In Figure 4 the mating surfaces 16, 17 are conical surfaces. In Figure 3 the first connector body 13 is a male part.

Figure 5 shows a third embodiment. In this embodiment the first connector part is a female part. Figure 3 uses corresponding reference numbers as used in the previous figures.

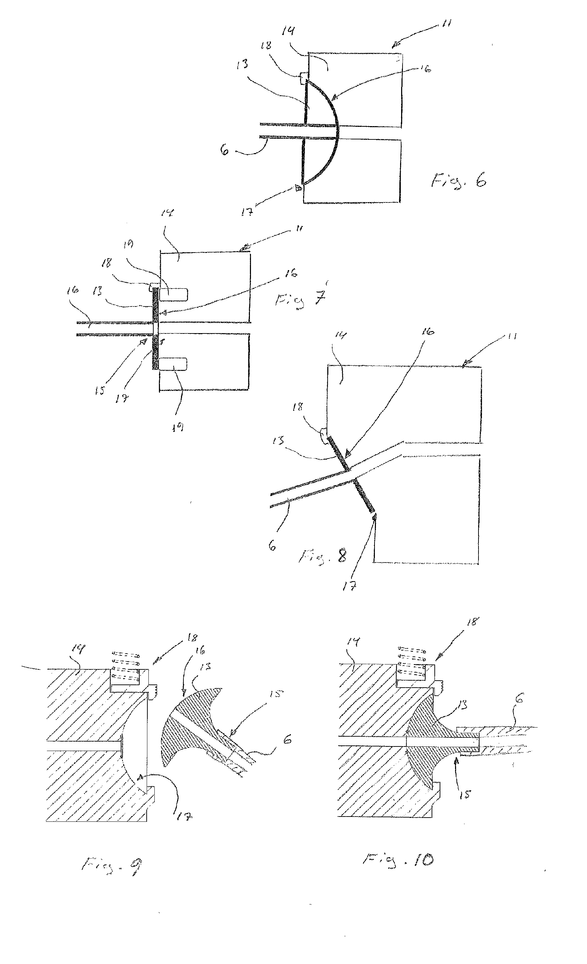

Figure 6 shows a fourth embodiment. In this embodiment the mating surfaces 16, 17 are curvilinear surfaces. Figure 4 uses corresponding reference numbers as used in the previous figures. Also this embodiment can be materialized by forming for example the first connector body as a male or a female part.

The embodiments shown in Figures 3 ― 6 are structures in which the fluid line falls off from the hosting device unless deliberately secured as intended. This is achieved with mating parts that do not protrude to each other deep enough to provide support for the mating parts to stay together protruded unless the connection is deliberately secured. The mating surfaces may have small protrusive part in order to achieve correct positioning but said protrusion is so small that it does not form any support.

In other words Figures 3 ― 6 show connector structures in which the fluid line falls off from the hosting device unless deliberately secured as intended. This is achieved with mating parts that have flat, tilted or rounded mating surfaces that do not provide support for a friction based loose connection. Any incomplete interfacing would lead into the gas sampling line falling off.

The functionality of the fluid line falling off from the detector unless secured can be boosted further by incorporating a force between the interfaces. Said force can be created by using appropriate resilient elements 19 as shown in Figure 7. Figure 6 shows a fifth embodiment and uses corresponding reference numbers as used in the previous figures.

The resilient elements 19 may be for example spring elements.

By having the care giver to deliberately work against the spring before a secure connection is made further strengthens the functionality of the designs. A spring force can be generated between the parts in several different ways. These would include (but not be limited to) having a soft compressible part between the interfaces. The compressible part could locate either in the fluid line side or on the fluid analyzer side. A spring force could also be generated by having bending pieces incorporated to the design.

Another means to further enhance the functionality of the connectors falling off unless secured is to make the design more susceptible for gravitation. Typically, gas sample connectors are placed on the sidewall of a monitoring device and hence the movement required for connecting the pieces happens in a horizontal plane. However, if the gas detector connector is tilted towards the ground or even positioned to point directly downwards there would be less friction to keep the pieces together than when having a horizontal connection. Figure 8 shows a sixth embodiment in which the connector is tilted towards the ground as described above. Figure 8 uses corresponding reference numbers as used in the previous figures.

The principles of the embodiments dscribed above can be applied to several different basic connector types such as a bayonnette connector, a conical connector with a thread, groove, etc.

Figures 9 and 10 show a seventh embodiment. Figures 9 and 10 use corresponding reference numbers as used in the previous figures. The embodiment shown in Figures 9 and 10 uses the principle shown in Figure 6, i.e. curvilinear mating surfaces 16, 17. Figures 9 and 10 show also that the locking mechanism 18 is a mechanism loaded by a reslient locking element. Said resilient locking element can be for example a spring element as shown in Figures 9 and 10 or any appropriate compressible part of resilient material.

The embodiments described above relate to the connection between the sampling tube 6 and the analyzer. This is not however the only possibility but the embodiments described can also be used for example as a connector structure between the sampling tube 6 and patient breathing tubing 3.

Sampling tube 6 can be provided with the first connector body 13 at one end or both ends thereof, or alternatively with the first connector body 13 at one end and the second connector body at the other end. It is also quite possible that the sampling tube 6 is provided with the second connector body 14 at both ends thereof.

This written description uses examples to disclose the invention, including the best mode, and also to enable any person skilled in the art to practice the invention, including making and using any devices or systems and performing any incorporated methods. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

## Claims

1. Connector structure for a fluid tube the connector structure comprising a first connector body (13) connected to a fluid tube (6), and a second connector body (14), the first connector body and the second connector body being adapted to be connectable to each other and provided with mating surfaces to create a fluid tight seal between the first connector body (13) and the second connector body (14), **characterized in that** the mating surfaces (16, 17) comprise only vertical and/or oblique parts and the connector structure is provided with a locking mechanism (18) and that the mating surfaces (16, 17) have been arranged to press tightly against each other and form a fluid tight seal with the locking mechanism (18) and the first and the second connector body (13, 14) are arranged to detach from each other without correct use of the locking mechanism (18).

2. The connector structure as claimed in claim **1, characteriz** e**d** in that the mating surfaces (16, 17) are flat surfaces.

3. The connector structure as claimed in claim **1, characteriz e**d in that the mating surfaces (16, 17) are conical surfaces.

4. The connector structure as claimed in claim **1, characteriz e** in that the mating surfaces (16, 17) are curvilinear surfaces.

5. The connector structure as claimed in claim 1 or 2, **character** i **zed** in that the mating surfaces (16, 17) are formed without substantially protruding components.

6. The connector structure as claimed in any of the claims 1 - 5, **c**h **aracterized** in that the connector structure is provided with resilient elements (19) to boost the fall of the first or the second connector body (16, 17).

7. The connector structure as claimed in claim **6, characteriz ed** in that the resilent elements (19) are spring elements.

8. The connector structure as claimed in any of the claims 1 ― **7,** c h **a**r**acter**i**e** d in that the locking mechanism (18) is a mechanism loaded by a resilient locking element.

9. The connector structure as claimed in claim **8, characteriz** e d in that the resilient locking element is a spring element.

10. The connector structure as claimed in any of the claims 1 ―**, c haracterized** in that the fluid tube (6) is a sample tube of a patient respiratory gas tubing (3).

11. The connector structure a s claimed in any of the claims 1 ― 10, c h a r a c t e r i z e d in that the second connector body (14) is a part of a gas analyzer (11).

12. The connector structure as claimed in any of the claims 1 ―0, c h a r a c t e r i z e d in that the second connector body (14) is a part of the patient respiratory tubing (3).

13. Sampling tube of a patient respiratory gas tubing, character i z e d in that at least one end of the sampling tube (6) is provided with the first connector body (13) of the connection structure as claimed in any of the claims 1 ― 12.

14. Sampling tube of a patient respiratory gas tubing, character i z e d in that at least one end of the sampling tube (6) is provided with the second connector body (14) of the connection structure as claimed in any of the claims 1 ―2.

15. The sampling tube of a patient respiratory gas tubing as claimed in claim 13 or 14, c h a r a c t e r i z e d in that the both ends of the sampling tube (6) is provided with the first connector body (13) or the second connector body (14).

## Amended claims

### Amended claims in accordance with Rule 137(2) EPC.

**1.** Connector structure for a fluid tube the connector structure comprising a first connector body (13) connected to a fluid tube (6), and a second connector body (14), the first connector body and the second connector body being adapted to be connectable to each other and provided with mating surfaces to create a fluid tight seal between the first connector body (13) and the second connector body (14), **characterized in that** the mating surfaces (16, 17) comprise only vertical and/or oblique parts and the connector structure is provided with a locking mechanism (18) and that the mating surfaces (16, 17) have been arranged to press tightly against each other and form a fluid tight seal with the locking mechanism (18) and the first and the second connector body (13, 14) are arranged to detach by the effect of gravity force from each other without correct use of the locking mechanism (18).

**2.** The connector structure as claimed in claim 1, **characteri**z**ed** in that the mating surfaces (16, 17) are flat surfaces.

**3.** The connector structure as claimed in claim 1, **characteri**z**ed** in that the mating surfaces (16, 17) are conical surfaces.

**4.** The connector structure as claimed in claim 1, **characteri**z**ed** in that the mating surfaces (16, 17) are curvilinear surfaces.

**5.** The connector structure as claimed in claim 1 or 2, **characterized in that** the mating surfaces (16, 17) are formed without substantially protruding components.

**6.** The connector structure as claimed in any of the claims 1 - 5, **characterize**d in that the connector structure is provided with resilient elements (19) to boost the fall of the first or the second connector body (16, 17).

**7.** The connector structure as claimed in claim 6, **characterized in that** the resilent elements (19) are spring elements.

**8.** The connector structure as claimed in any of the claims 1 - 7, **characterie**d in that the locking mechanism (18) is a mechanism loaded by a resilient locking element.

**9.** The connector structure as claimed in claim 8, **characterized in that** the resilient locking element is a spring element.

**10.** The connector structure as claimed in any of the claims 1 - 9, **characterize**d in that the fluid tube (6) is a sample tube of a patient respiratory gas tubing (3).

**11.** The connector structure a s claimed in any of the claims 1 - 10, **characterized in that** the second connector body (14) is a part of a gas analyzer (11).

**12.** The connector structure as claimed in any of the claims 1 - 10, **characterized in that** the second connector body (14) is a part of the patient respiratory tubing (3).

**13.** Sampling tube of a patient respiratory gas tubing, **characterized in that** at least one end of the sampling tube (6) is provided with the first connector body (13) of the connection structure as claimed in any of the claims 1-12.

**14.** Sampling tube of a patient respiratory gas tubing, **characterized in that** at least one end of the sampling tube (6) is provided with the second connector body (14) of the connection structure as claimed in any of the claims 1 - 12.

**15.** The sampling tube of a patient respiratory gas tubing as claimed in claim 13 or 14, **characterized in that** the both ends of the sampling tube (6) is provided with the first connector body (13) or the second connector body (14).
